Europäisches Patentamt

European Patent Office

Office européen des brevets

(19)

(11) **EP 0 798 388 B1**

(12) **EUROPEAN PATENT SPECIFICATION**

(45) Date of publication and mention
of the grant of the patent:
**12.06.2002 Bulletin 2002/24**

(51) Int Cl.⁷: **C12Q 1/68**, G01N 33/543,
G01N 33/577, G01N 21/47

(21) Application number: **97104404.5**

(22) Date of filing: **14.03.1997**

(54) **Method for detecting a mutation in a gene**

Methode zum Nachweis einer Mutation in einem Gen

Méthode de détection d'une mutation dans un gène

(84) Designated Contracting States:
**DE FR GB IT**

(30) Priority: **14.03.1996 JP 5730296
24.02.1997 JP 3892997**

(43) Date of publication of application:
**01.10.1997 Bulletin 1997/40**

(73) Proprietor: **Sysmex Corporation
Hyogo (JP)**

(72) Inventor: **Fukuda, Shigehiro
Osaka-shi, Osaka (JP)**

(74) Representative: **Kolb, Helga, Dr. Dipl.-Chem. et al
Hoffmann Eitle,
Patent- und Rechtsanwälte,
Arabellastrasse 4
81925 München (DE)**

(56) References cited:
**EP-A- 0 159 719        EP-A- 0 324 474
US-A- 5 104 791**

- **DATABASE WPIL, AN 94-251 710, DERWENT
  PUBLICATIONS LTD., London; & JP-A-06 181
  800 (AISIN SEIKI KK)**
- **DATABASE WPIL, AN 93-260 875, DERWENT
  PUBLICATIONS LTD., London; & JP-A-05 176
  800 (BIOSENSOR KENKYUSHO KK)**

**Description**

This invention relates to a method for detecting an amplified gene which is widely usable in the fields of immunology, molecular biology, diagnostics and the like.

With the recent progress in genetic engineering, there have been devised many methods for largely amplifying genes in trace amount. Use of these methods makes it possible to amplify a gene contained in a trace amount in a sample. PCR (polymerase chain reaction) is the most famous one among the gene amplification methods. In this method, gene fragments (primers) complementary to the sequences at the 3'- and 5'-ends of the region containing the target gene are constructed. Then these primers are mixed with a sample together with bases which are constituents of genes and the target gene is synthesized under the action of an enzyme synthesizing the gene (DNA polymerase). In this method, the following three reactions are repeated in 20 to 40 cycles in general: (1) dissociation of two strands of DNA by thermal denaturation; (2) annealing with the primers; and (3) synthesis of complementary strands by the DNA polymerase. In the step (1), the two strands of the double-stranded DNA are separated into single strands by heating. When a DNA polymerase originating in a thermostable microorganism is employed therein, it is unnecessary to supply fresh DNA polymerase in each cycle. Thus the gene can be amplified by repeating the reactions while merely changing temperature. By using this method, the target gene can be amplified a billion-fold or million-fold.

In these days, a number of instruments called thermal cyclers, with which the amplification process can be automatically carried out, are marketed and used in the fields of medicine, diagnostics, molecular biology and the like.

As described above, there have been already established methods for conveniently amplifying genes. However, these genes thus amplified are still detected by electrophoresis in many cases. It takes a lot of time and labor to perform this procedure which comprises electrophoresing a sample on a gel made of, for example, polyacrylamide and then staining to thereby examine the presence of the target gene. Although the gene amplification per se can be completed within several hours, a long time is required for detecting the gene thus amplified. Therefore, it takes two or three days in total to detect the gene. In addition, electrophoresis is unsuitable for dealing with many specimens. One of the reasons for little diffusion of gene diagnosis in the field of clinical diagnosis resides in that it is troublesome to detect genes. In recent years, attempts are made to detect amplified genes by HPLC (high performance liquid chromatography) or capillary electrophoresis. However, these methods are also unsuitable for dealing with many specimens.

Further, it is attempted to detect a gene by the particle agglutination method and there have been disclosed techniques therefor. For example, USP No. 5,104,791 discloses the following process. Two gene fragments complementary to the target gene are prepared and bonded to particles. Next, they are bonded to the target gene so as to cause agglutination. Then the presence and amount of the target gene are assayed by measuring the signal due to the agglutination.

However, the method of USP No. 5,104,791 suffers from a problem that two different gene fragments should be bonded to particles for each target gene. In addition, the target gene should be a single-stranded one, since gene fragments complementary thereto are employed in this method. However, a DNA usually occurs in the double-stranded state and a double-stranded gene is also obtained after the completion of the gene amplification. In this method, therefore, it is needed to dissociate the gene into a single-stranded one after the completion of the amplification. After the dissociation into single strands, the sample still contains the complementary gene which undergoes the reaction in competition with the gene fragments on the particles. This phenomenon is not desirable in the assay method, since the detection sensitivity is lowered thereby.

Further, EP A 0 159 719 discloses a hybridisation method for detection of genetic materials. The method described therein is based on utilizing two labelled single stranded polynucleotides segments which are complementary to the same or the opposite strands of the target genetic material. The methods result in the formation of a double hybrid and/or a multihybrid. That means, at least two labels have to be detected.

On the other hand, it is theoretically possible to detect a gene with the use of an antibody capable of recognizing the target gene. Although a gene might be detected depending on the agglutination of particles by using this method, it seems very difficult to perform this method. A gene carries genetic information conserved in a sequence consisting of bases of four types. That is, a gene is composed of nothing but these bases of four types and it is highly difficult to construct an antibody reacting exclusively with a certain base sequence. An antibody recognizing a certain gene sequence would also react with a part of another gene sequence closely similar thereto. This phenomenon, which is called "cross-reaction", brings about a serious problem with respect to antibodies recognizing genes. In this method, it is also required to prepare particles to which at least two antibodies have been bonded for each gene. Thus it cannot be regarded as a convenient method.

It is well known that some genes have mutations which sometimes closely relate to diseases.

There have been known a number of diseases which are caused by a mutation in the gene or in which a mutation in the gene serves as a risk factor. Examples of these diseases include those which have been known for a long time such as thalassemia and hemophilia and those which have been revealed recently such as familial amyotrophic lateral sclerosis and familial Alzheimer's disease. Some of these diseases are induced merely by a mutation in a part of the gene or even in a base in the base sequence, i.e., so-called point mutation. To detect such a point mutation, use is made of the DGGE, SSCP and RFLP methods today. In the DGGE (denaturant concentration gradient gel electrophoresis) method, electrophoresis is performed on a gel with a denaturant concentration gradient by taking advantage of the fact that the stability of a double-stranded gene is altered when a point mutation arises. In the SSCP (single-stranded DNA conformational polymorphism) method, on the other hand, detection is effected on the basis of the principle that when an amplified double-stranded gene is dissociated into single strands and electrophoresed on an undenatured polyacrylamide gel, these strands show different mobilities, since each single-stranded DNA has a specific conformation depending on its base sequence. Use of this method makes it possible to detect a mutation in a single base, even among several hundred bases, as a difference in mobility.

A mutation occurring at a definite position can be sometimes detected by The RFLP (restriction enzyme fragment length polymorphism) method. In this method, use is made of a phenomenon that a mutation arising at a definite position makes the position as the cleavage site of a certain restriction enzyme or prevents a normal cleavage site from breakage. A gene is amplified on the both sides of its mutated site. Then the amplification product thus obtained is treated with a certain restriction enzyme. Thus the cleavage site appears or disappears depending on the occurrence of the mutation. In the RFLP method, these changes are detected through electrophoresis.

These methods are so elaborated that a mutation even at a single base can be detected thereby. However, each of these methods involves electrophoresis as the major means for the detection and, therefore, is not so excellent in quickness, convenience and suitability for dealing with many specimens.

It is an object of the present invention to provide a method for conveniently and quickly detecting an amplified gene.

It is another object of the present invention to provide a safe and sensitive detection system without using any isotope.

Further, it is another object of the present invention to provide a method for conveniently and quickly detecting a mutation contained in an amplified gene.

Under these circumstances, the present inventors have conducted extensive studies. As a result, they have successfully established a method for detecting an amplified gene by taking advantage of agglutination which is induced by binding an antigen or an antibody to gene fragments to be used in the amplification and using particles having an antibody or an antigen recognizing the above-mentioned antigen or antibody bonded thereto, or particles having a substance specifically binding to the antigen or antibody bonded onto the surface thereof.

The present inventors have further found out that, when the amplified gene has a mutation in the above-mentioned gene detection method, the occurrence of the cleavage caused by reacting with a restriction enzyme can be detected as the degree of agglutination of the particles and thus the mutation in the gene can be detected.

Accordingly, the present invention provides a method for detecting a mutation in a gene comprising, in a method for detecting a gene comprising reacting a double-stranded gene, which has been amplified with the use of gene fragments having an antigen or an antibody bonded thereto, with particles having an antibody or an antigen recognizing the above-mentioned antigen or antibody bonded thereto or particles having a substance specifically binding to the above-mentioned antigen or antibody bonded onto the surface thereof, and measuring the degree of agglutination of the particles to thereby detect the target gene;

when the gene to be used in the above amplification has a mutation site, treating it with a restriction enzyme the cleavage manner of which varies depending on the presence of the mutation site, thus allowing the restriction enzyme to selectively cleave the gene, and detecting the change in the degree of agglutination of the particles thus induced to thereby detect the mutation in the gene.

Fig. 1 is a schematic view showing the gene detection method of the present invention.

Fig. 2 gives an example of the particle size distribution determined by using a fully automatic immunoassay system.

PRINCIPLE OF THE GENE DETECTION METHOD OF THE INVENTION

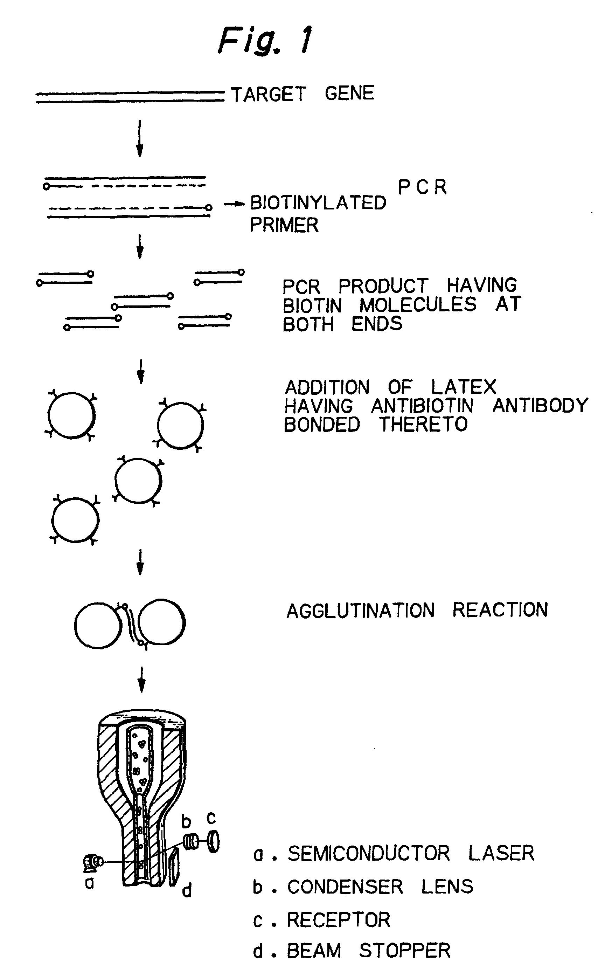

The principle of the gene detection method used in the present invention is as follows. Fig. 1 is a schematic view showing the gene detection method of the present invention.

First, an antigen or an antibody is preliminarily bonded to the gene fragments to be used as the starting material in the amplification. Then gene amplification is carried out in some manner by using the gene fragments as primers. When the sample contains the target gene, the target gene is produced in a large amount by gene amplification starting from the gene fragments having the antigen or antibody bonded thereto which have been added above. Most of the genes thus amplified are constructed from the gene fragments having the antigen or antibody bonded thereto and therefore carry the antigen or antibody bonded to both ends. The binding site of the antigen or antibody is not restricted to the ends of the gene but one molecule thereof may be bonded at any site. When it is bonded to an end, the binding site is on the opposite side to the direction of the amplification.

When particles having an antibody or an antigen with affinity for the above-mentioned antigen or antibody bonded thereto are added to the sample after the completion of the amplification, these particles react with the amplified gene and undergo agglutination. Although the agglutination thus induced can be observed with the naked eye or analyzed by determining turbidity, absorbance, etc., the degree of agglutination can be determined by passing the particles through a sheath flow, irradiating each particle with laser beams and measuring the intensity of the scattered light. By using this procedure, the amplified gene can be detected at a high sensitivity. Also, it is possible to use the electric resistance method for the determination by adequately controlling the particle size. When troubles such as clogging in the detector are taken into consideration, it is preferable to select an optical method for the determination. The agglutinating particles can be quickly and accurately detected by optically masuring the size of the agglutinating latex particles with the use of a fully automatic immunoagglutination assay system (for example, PAMIA-30 manufactured by TOA MEDICAL ELECTRONICS CO., LTD.).

APPLICATION TO THE DETECTION OF MUTATION

In the method for detecting a mutation in a gene according to the present invention, an antigen is bonded to gene fragments to be used as the starting material in the gene amplification and then gene amplification is performed in some manner by using the gene fragments. When the sample contains the target gene having a mutation, the target gene is produced in a large amount by the gene amplification starting from the gene fragments having the antigen bonded thereto which have been added above. Most of the genes thus amplified are constructed from the gene fragments having the antigen bonded thereto and therefore carry the antigen bonded to both ends. The amplified gene contains the mutation.

When particles having an antibody with affinity for the above-mentioned antigen bonded thereto are added to the sample after the completion of the amplification, these particles react with the amplified gene and undergo agglutination. Although the agglutination thus induced can be observed with the naked eye or analyzed by determining turbidity, absorbance, etc., the degree of agglutination can be determined by passing the particles through a sheath flow, irradiating each particle with laser beams and measuring the intensity of the scattered light as described above.

If the amplified gene has a mutation and thus shows a cleavage site of a certain restriction enzyme which is never observed in the normal specimen, then the amplified gene is treated with the restriction enzyme. When the amplified gene is thus cleaved, the degree of agglutination of the particles is lowered. When it is not cleaved, on the other hand, no change is observed in the agglutination. In this case, a cleavage site of the restriction enzyme is newly formed due to the mutation. On the contrary, it is observed in some cases that the gene cannot be cleaved with a restriction enzyme at a specific site where it is cleaved in a normal specimen. Accordingly, the occurrence of the mutation in the gene can be judged by, after the completion of the gene amplification, preparing a sample treated with the restriction enzyme and another one untreated therewith and examining the agglutination reaction in both of these samples.

The restriction enzyme may be added either before or after the reaction with the particles having the antibody bonded thereto. However, it is advantageous to add the restriction enzyme before the reaction with the particles, since a marketed apparatus for analyzing particle agglutination (for example, PAMIA-30 manufactured by TOA MEDICAL ELECTRONICS CO., LTD.) can be used almost as such in this case.

The mutations detectable by the method of the present invention involve many ones which have been detected by the conventional RFLP method such as those in association with familial amyotrophic lateral sclerosis, familial hypercholesterolemia, erythroenzymopathy and hemophilia. By using the method of the present invention, gene diagnosis can be performed very conveniently, compared with the conventional RFLP method wherein a gene is detected by electrophoresis.

BEST MODE FOR CARRYING OUT THE INVENTION

Although various antigens or antibodies are usable as the antigen or antibody to be bonded to the gene fragments complementary to the sequences at the 3'- and 5'-ends of the region containing the target gene, one having a lower molecular weight would the less interfere the gene amplification. From this viewpoint, it is preferable to use a hapten which is a low-molecular weight antigen. Examples of the hapten include biotin and FITC (fluorescein isothiocyanate).

Now, description will be made on a case where gene fragments having an antigen bonded thereto are employed.

The gene fragments having an antigen bonded thereto can-be constructed by a method publicly known in the art. For example, a biotinylated primer may be synthesized from a biotinylated nucleotide (BIOTIN dX™, available from Wako Pure Chemical Industries, Ltd.) and a usual nucleotide with the use of an automatic synthesizer (manufactured by ABI). As described above, it is preferable that biotin molecules are bonded to the 3'- and 5'-ends. However, it may be bonded midway in the primer without restriction, so long as the subsequent amplification is not inhibited thereby. Moreover, a biotin molecule may be bonded to an end of an oligonucleotide by using Oligonucleotide Biotin Labeling Kit (manufactured by Amersham) or the like. It is also possible to entrust Takara Shuzo Co., Ltd. with the synthesis of desired biotinylated primers.

By using the thus obtained gene fragments having an antigen bonded thereto as primers, a gene is amplified by the PCR method, etc. The gene amplification may be performed by a method publicly known in the art. For example, the antigen-bonded primers, a nucleotide mixture and DNA polymerase are subjected to PCR by using a thermal cycler (manufactured by Perkin-Elmer) to thereby give a double-stranded gene.

Separately, particles, to which an antibody capable of recognizing the above-mentioned antigen or a substance binding specifically to the above-mentioned antigen has been bonded, are prepared.

Various particles are usable therefor. When the particles are to be passed through a sheath flow and analyzed, however, it is important that they have a uniform particle size. From this viewpoint, latex particles are seemingly the most useful ones. The particle size is not particularly restricted, so long as the degree of agglutination can be detected. For example, use can be made of a soap-free polystyrene latex (particle size: 0.8 $\mu$m) available from Sekisui Chemical Co., Ltd.

As the antibody to be bonded onto the surface of the particles, various ones are usable. In the preferred embodiment of the present invention, the amplified gene carries the same antigen at both ends. Thus, it is possible to use a monoclonal antibody against this antigen. That is to say, a single monoclonal antibody, which cannot be used alone in the agglutination method usually, can induce the agglutination of the particles in this method.

In the method of USP No. 5, 104,791 as cited above, two gene fragments complementary to the target gene are bonded to the target gene so as to induce agglutination. However, this method suffers from a problem that two different gene fragments should be bonded to the particles for each target gene. In contrast thereto, the method of the present invention is advantageous in that antibody-bonded particles of only one type are needed in the detection, so long as the same labeling antigen is employed. It is a great advantage that any gene can be detected by using particles of only one type.

Moreover, the antibody to be used in the present invention is not the one against the target gene but arbitrarily selected. Thus, an antigen having a high specificity or one which would never invade into the sample to be assayed may be used therefor, which makes it possible to provide an assay system by which the aimed reaction can be certainly effected at a high sensitivity.

In the method of the present invention, use can be made of not only an antibody but a substance binding specifically to the antigen employed in the present invention. For example, it is possible to utilize the specific bond between biotin/avidin or streptoavidin, ligand/receptor, sugar chain/lectin or enzyme/inhibitor. In such a case, one is bonded to the primers while another is bonded to the particles. It is necessary that the primers having the antigen bonded thereto would never interfere the gene amplification.

As described above, the method of the present invention is advantageous in that any target gene can be detected by using antibody-bonded particles of only one type, so long as the same antigen is bonded to the primers. Since the antibody carried on the particles binds to the antigen bonded to the gene fragments, any gene can be detected with the use of particles of a single type. Alternatively, the antigens bonded to the primers of the 3'- and 5' -ends may be different from each other. In this case, it is necessary to prepare particles having the antibodies against both antigens. Therefore, it is preferable that a single antigen is bonded to both primers in order to simplify the constitution of the reagent.

An antibody or a substance binding specifically to the antigen may be bonded to the particles by a method publicly known in the art, for example, physical adsorption, covalent binding, or the like. Among all, the physical adsorption method can be easily performed. In general, it can be carried out by mixing an antibody solution with a particle suspension and reacting them at 4°C overnight or at 25°C for several hours.

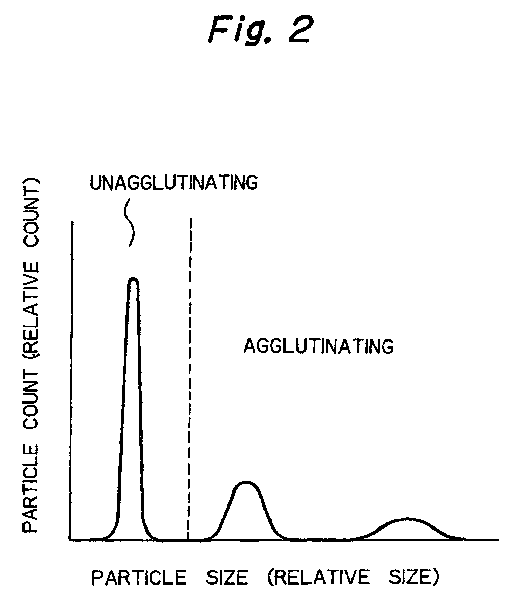

When the double-stranded gene thus amplified is reacted with the particles to which an antibody recognizing the above-mentioned antigen or a substance binding specifically to the above-mentioned antigen has been bonded, the antigen/antibody reaction causes the agglutination of the particle to thereby give dimers, trimers, tetramers, etc. The degree of agglutination can be evaluated by passing individual particles, either agglutinating or not, through a sheath flow cell, irradiating these particles with laser beams and then measuring the intensity of the scattered light. This procedure can be easily and quickly carried out by using, for example, the fully automatic immunoassay system as described above. Fig. 2 shows by way of example the particle size distribution determined by using such a fully automatic immunoassay system. The formation of the agglutinates indicates that the target gene is contained in the sample. By using the method of the present invention, the presence and amount of the target gene can be easily determined.

The target gene which can be detected by the gene detection method of the present invention involves not only DNAs but also RNAs. When the target gene is an mRNA, for example, gene amplification may be effected after preliminarily constructing a cDNA with the use of a reverse transcriptase.

Use of the method of the present invention makes it possible to conveniently and quickly detect an amplified gene. The significant advantage of the present invention resides in that, when particles to which an antibody recognizing an antigen or a substance binding specifically to the antigen has been bonded are preliminarily prepared, the presence and amount of any target gene can be determined by using these particles. Further, the gene detection method of the present invention is safe, since no isotope is used therein. Because of being applicable not only to DNAs but also to RNAs, the gene detection method of the present invention is highly useful in the diagnosis of many diseases including AIDS and hepatitis, in particular, in quickly diagnosing many samples.

Moreover, use of the method for detecting a mutation in a gene according to the present invention makes it possible to detect the mutation within a very short period of time, compared with the detection by the conventional methods such as RFLP method.

To further illustrate the present invention in greater detail, and not by way of limitation, the following Examples will be given.

[Examples]

Example 1: Detection of human genomic DNA via the detection of PCR product with the use of biotinylated primers:

PREPARATION OF BIOTINYLATED PRIMERS

DNA of the b-globulin region in human genomic DNA [Saiki, R.K. et al., (1988) Science, 239, 487-491] was amplified and the amplification product was detected via the agglutination of latex.

The sample subjected to the detection was human leukocytes. As the primers, primers having the same base sequences as those of primers PC03 and KM38, which were contained in a β-globulin primer set marketed from Takara Shuzo Co., Ltd., and each carried one biotin molecule bonded to the end thereof were synthesized and employed as biotinylated primers. The gene fragment amplified by PC03 and KM38 was 167 bp in size.

The base sequences of the primers thus constructed were as follows:

**PC03-Biotin: Biotin-ACACAACTGTGTTCACTAGC; and**

**KM38-Biotin: TGGTCTCCTTAAACCTGTCTTG-Biotin.**

These biotinylated primers were synthesized by using an automatic synthesizer (manufactured by ABI).

PREPARATION OF SAMPLE

Human blood was employed as the sample. Blood was collected with the use of trisodium citrate as an anticoagulant and centrifuged at 3,000 rpm for 15 minutes and then the buffy coat was taken up. The buffy coat was then diluted with physiological saline to thereby give samples containing about 100000, 10000 and 1000 leukocytes per 10 μl. The leukocytes were counted by using an blood cell counter Model F-800 (manufactured by TOA MEDICAL ELECTRONICS CO., LTD.). Since most of all DNAs in blood originate in leukocytes, DNA samples to be used in PCR were prepared therefrom by the guanidium isothiocyanate method [Bunshi Saibo Seibutsugaku Kiso Jikkenho (Basic Techniques for Experiments in Molecular Cytological Biology), Nankodo].

GENE AMPLIFICATION

PCR was carried out by using a thermal cycler (manufactured by Perkin-Elmer).

The composition of the PCR solution was as follows:

| | |
|---|---|
| buffer (200 mM Tris buffer, pH 8.4, 15 mM of MgCl$_2$, 1 mg/ml of BSA) | 5 μl |
| purified water | 39.5 μl |
| 10 mM dNTP mixture | 1 μl |
| biotinylated primers (each 10 μM) | each 1 μl |
|     PC03-Biotin : | |
|     KM38-Biotin : | |

(continued)

| TaqDNA polymerase (AmpliTaq™, manufactured by Takara Shuzo Co., Ltd.) | 0.5 μl |
|---|---|
| extracted DNA solution | 2 μl. |

PCR was carried out in 20 cycles each consisting of 45 seconds at 94°C, 25 seconds at 55°C and 3 minutes at 72°C.

PREPARATION OF LATEX HAVING ANTIBIOTIN ANTIBODY BONDED THERETO

A dispersion of a polystyrene latex (particle size: 0.78 μm, manufactured by Sekisui Chemical Co., Ltd.) in 10 mM PBS (pH 7.0) was adjusted to a concentration of 5 % (w/v). Then antibiotin antibody (monoclonal antibody, manufactured by Bio-Design) was added thereto at a ratio of 50 μg per ml of the latex dispersion and the mixture was reacted at 4 °C for 24 hours. After centrifuging at 12,000 rpm for 10 minutes, 0.1 M PBS containing 1 mg/ml of BSA was added in the same amount as added above and the particles were dispersed therein. After dispersing in the same buffer by centrifuging again, a latex having the biotin antibody bonded thereto was obtained.

AGGLUTINATION REACTION

After the completion of PCR, 10 μl of the antibiotin antibody-bonded latex particles (0.5 %) were added to 10 μl of the PCR sample. Next, 80 μl of a 0.1 M phosphate buffer containing 1 mg/ml of BSA was further added thereto and the mixture was reacted at 45°C for 15 minutes. Then the degree of agglutination of the latex particles was determined. In the reaction and determination, a fully automatic immunoagglutination assay system PAMIA-30 (manufactured by TOA MEDICAL ELECTRONICS CO., LTD.) was used and the intensity of the forward scattered light was measured.

The degree of agglutination is expressed in the ratio (%) of the count of the agglutinating particles to the total count of the particles. Table 1 shows the results. As Table 1 clearly shows, a sufficient degree of agglutination was achieved even in the sample containing 1000 leukocytes per 10 μl. The sample free from any DNA originating in leukocyte [namely, the one having the composition of the PCR solution but containing no extracted DNA (DNA of leukocyte)] showed no agglutination of the latex, while the samples containing leukocyte DNA showed agglutination. These facts indicate that the latex reagent underwent agglutination by reacting with the biotin in the PCR product.

| Leukocyte count (/10 μl) | 100000 | 10000 | 1000 | 0 |
|---|---|---|---|---|
| Agglutination ratio (P/T %) | 43.2 | 40.5 | 35.9 | 3.4 |

Example 2: Detection of mutation in human hemoglobin gene:

By using the method of the present invention, a mutation in human hemoglobin gene was detected.

PREPARATION OF BIOTINYLATED PRIMERS

As the primers, use was made of those having the same base sequences as the ones contained in a β-globulin primer set marketed from Takara Shuzo Co., Ltd. This set contains six primers with different sites and fragments of nine sizes can be amplified therewith by appropriately combining these primers.

In this Example, primers having the same base sequences as PC03 and PC04 were synthesized and one biotin molecule was bonded to the end of each primer to thereby give a biotinylated primer. The gene fragment amplified by PC03 and PC04 was 110 bp in size.

The base sequences of the primers thus constructed were as follows:

**PC03-Biotin: Biotin-ACACAACTGTGTTCACTAGC; and**

**PC04-Biotin: CAACTTCATCCACGTTCACC-Biotin.**

Takara Shuzo Co., Ltd. was entrusted with the synthesis of desired biotinylated primers.

PREPARATION OF SAMPLE

The mutation detectable by combining the above-mentioned primers is codon 17 mutant allele.

This mutation, in which A is replaced by T in the nonsense region, can be detected by the RFLP method with the use of a restriction enzyme Nhe I. Namely, in a normal specimen, the PCR product obtained by the combination of the above-mentioned primers is not cleaved with Nhe I. Therefore, only one band is detected at 110 bp in electrophoresis following the enzyme reaction. On the other hand, a specimen carrying this mutation has a cleavage site of Nhe I and thus shows a band at 87 bp in electrophoresis. A hetero specimen shows both of these bands.

The samples were prepared in accordance with the method described in Clin. Biochem., 26, 497-503 (1993). Normal human blood and one having been proved to carry the mutation at the above-mentioned site were employed as the samples. Genomic DNA was extracted by using RapidPrep Genomif DNA Isolation Kits for Blood (manufactured by Pharmacia) to thereby give samples to be used in PCR.

GENE AMPLIFICATION

PCR was carried out by using a thermal cycler (manufactured by Perkin-Elmer) and a reagent GeneAmp (manufactured by Takara Shuzo Co., Ltd.).

The composition of the PCR solution was as follows:

| | |
|---|---|
| buffer (200 mM Tris buffer, pH 8.4, 15 mM of MgCl$_2$, 1 mg/ml of BSA) | 5 μl |
| purified water | 39.5 μl |
| 10 mM dNTP mixture | 1 μl |
| biotinylated primers (each 10 μM) | each 1 μl |
| TaqDNA polymerase | 0.5 μl. |

PCR was carried out in 35 cycles each consisting of 1 minute at 94°C, 2 minutes at 55°C and 2 minutes at 68°C.

PREPARATION OF LATEX HAVING ANTIBIOTIN ANTIBODY BONDED THERETO

A dispersion of a polystyrene latex (particle size: 0.78 μm) in 20 mM Tris-buffer (pH 7.0) was adjusted to a concentration of 5 % (w/v). Then antibiotin antibody (monoclonal antibody, manufactured by Bio-Design) was added thereto at a ratio of 50 μg per ml of the latex dispersion and the mixture was reacted at 4°C for 24 hours. After centrifuging at 12,000 rpm for 10 minutes, 0.1 M PBS containing 1 mg/ml of BSA was added in the same amount as added above and the particles were dispersed therein. After dispersing in the same buffer by centrifuging again, a latex having the biotin antibody bonded thereto was obtained.

AGGLUTINATION REACTION

First, it was examined whether or not the gene amplification had been completed by PCR in the following manner. After the completion of PCR ,10 μl of the antibiotin antibody-bonded latex particles (0.5 %) were added to 10 μl of the PCR sample. Next, 80 μl of a 0.1 M phosphate buffer containing 1 mg/ml of BSA was further added thereto and the mixture was reacted at 45°C for 15 minutes. Then the degree of agglutination of the latex particles was determined. In the reaction and determination, a fully automatic immunoagglutination assay system PAMIA-30 (manufactured by TOA MEDICAL ELECTRONICS CO., LTD.) was used.

Next, the mutation was detected in the following manner. Similar to the above case, 10 μl portions of the sample were taken after the completion of PCR and 10 μl of the buffer and 10 μl of the buffer containing 10 U of Nhe I were respectively added thereto. Both of these samples were incubated at 37°C overnight and each reacted with the particles in the same manner as the one described above followed by the determination with PAMIA-30.

The degree of agglutination is expressed in the ratio (%) of the count of the agglutinating particles to the total count of the particles.

The results are as follows. In the normal specimen, the addition of the restriction enzyme caused no change in the degree of agglutination. In the specimen having the mutation at the codon 17, on the other hand, the sample not reacted with the enzyme showed no change in the degree of agglutination but the sample reacted with the enzyme showed a decrease in the degree of agglutination. These facts suggest that mutation caused the appearance of the cleavage site of Nhe I and thus the PCR product was cleaved by the restriction enzyme, thus lowering the degree of agglutination of the latex. As discussed above, it is indicated that a point mutation can be detected by using the latex agglutination reaction.

|  | Sample free from restriction enzyme | Sample containing restriction enzyme |
|---|---|---|
| Normal specimen | 21.3 % | 20.4 % |
| Specimen with mutation | 22.9 % | 5.6 % |

Agglutination ratio (P/T) %:

(count of agglutinating particles/total count of

particles) x 100.

## Claims

1. A method for detecting a mutation in a gene comprising, in a method for detecting a gene comprising reacting a double-stranded gene, which has been amplified with the use of gene fragments having an antigen or an antibody bonded thereto, with particles having an antibody or an antigen recognizing the above-mentioned antigen or antibody bonded thereto or particles having a substance specifically binding to the antigen or antibody bonded onto the surface thereof, and measuring the degree of agglutination of the particles to thereby detect the target gene; when the gene to be used in the above amplification has a mutation site, treating it with a restriction enzyme the cleavage manner of which varies depending on the presence of the mutation site, thus allowing the restriction enzyme to selectively cleave the gene, and detecting the change in the degree of agglutination of the particles thus induced to thereby detect the mutation in the gene.

2. A method as claimed in Claim 1 wherein said particles are latex with a uniform particle size.

3. A method as claimed in Claim 1 or 2, wherein the degree of agglutination is determined by passing the particles through a sheath flow cell, irradiating each particle with laser beams and measuring the intensity of the scattered light.

4. A method as claimed in Claim 3, wherein said scattered light is forward scattered light.

5. A method as claimed in Claim 1 wherein an antigen is bonded to the gene fragments while an antibody recognizing said antigen is bonded onto the surface of the particles.

6. A method as claimed in Claim 5, wherein said antigen is a hapten.

7. A method as claimed in Claim 5 or 6, wherein said antibody is a monoclonal antibody.

8. A method as claimed in any of Claims 5 to 7, wherein one antigen is bonded to the gene fragments and one monoclonal antibody is bonded onto the surface of the particles.

## Patentansprüche

1. Verfahren zum Nachweis einer Mutation in einem Gen, umfassend in einem Verfahren zum Nachweis eines Gens, das die Umsetzung eines doppelsträngigen Gens, das unter Verwendung von Genfragmenten mit daran gebundenem Antigen oder Antikörper amplifiziert wurde, mit Teilchen, die einen Antikörper oder ein Antigen aufweisen, die das oben erwähnte gebundene Antigen oder den Antikörper erkennen, oder mit Teilchen, die eine Substanz aufweisen, die spezifisch an das an die Oberfläche gebundene Antigen oder den Antikörper binden, und Messung des Grads der Agglutinierung der Teilchen, um dadurch das Zielgen nachzuweisen, umfasst; wenn das in der obigen Amplifizierung zu verwendende Gen eine Mutationsstelle aufweist, seine Behandlung mit einem Restriktionsenzym, dessen Spaltungsart variiert, abhängig von der Gegenwart der Mutationsstelle, wodurch dem Restriktionsenzym ermöglicht wird, das Gen selektiv zu spalten, und Nachweis der Veränderung im Grad der Agglutinierung der so induzierten Teilchen, um dadurch die Mutation in dem Gen nachzuweisen.

**2.** Verfahren gemäss Anspruch 1, wobei die Teilchen Latex mit einer gleichmässigen Teilchengrösse sind.

**3.** Verfahren gemäss Anspruch 1 oder 2, wobei der Grad der Agglutinierung bestimmt wird, indem die Teilchen durch eine Hüllflussdurchflusszelle geführt werden, jedes Teilchen mit Laserstrahlen bestrahlt wird und die Intensität des gestreuten Lichts gemessen wird.

**4.** Verfahren gemäss Anspruch 3, wobei das gestreute Licht nach vorwärts gestreutes Licht ist.

**5.** Verfahren gemäss Anspruch 1, wobei ein Antigen an die Genfragmente gebunden ist, während ein Antikörper, der das Antigen erkennt, an die Oberfläche der Teilchen gebunden ist.

**6.** Verfahren gemäss Anspruch 5, wobei das Antigen ein Hapten ist.

**7.** Verfahren gemäss Anspruch 5 oder 6, wobei der Antikörper ein monoklonaler Antikörper ist.

**8.** Verfahren gemäss einem der Ansprüche 5 bis 7, wobei ein Antigen an die Genfragmente gebunden ist und wobei ein monoklonaler Antikörper an die Oberfläche der Teilchen gebunden ist.

**Revendications**

**1.** Procédé pour détecter une mutation dans un gène, comprenant les étapes consistant à, dans un procédé pour détecter un gène qui comprend les étapes consistant à faire réagir un gène à double brin, qui a été amplifié à l'aide de fragments de gène auxquels sont liés un antigène ou un anticorps, avec des particules auxquelles sont liés un anticorps ou un antigène reconnaissant l'antigène ou l'anticorps mentionné ci-dessus, ou avec des particules présentant une substance se liant spécifiquement à l'antigène ou à l'anticorps lié à sa surface, et à mesurer le degré d'agglutination des particules pour ainsi détecter le gène visé;

lorsque le gène à utiliser dans l'amplification ci-dessus présente un site de mutation, le traiter avec une enzyme de restriction qui opère le clivage d'une manière qui varie en fonction de la présence du site de mutation, pour ainsi permettre à l'enzyme de restriction de cliver sélectivement le gène, et détecter la modification du degré d'agglutination des particules ainsi induites pour ainsi détecter la mutation du gène.

**2.** Procédé selon la revendication 1, dans lequel les dites particules sont un latex avec une taille uniforme de particules.

**3.** Procédé selon la revendication 1 ou 2, dans lequel le degré d'agglutination est déterminé en faisant passer les particules dans une cellule d'écoulement à fourreau, en irradiant chaque particule par des faisceaux laser, et en mesurant l'intensité de la lumière diffusée.

**4.** Procédé selon la revendication 3, dans lequel la dite lumière diffusée est de la lumière diffusée vers l'avant.

**5.** Procédé selon la revendication 1, dans lequel un antigène est lié aux fragments de gène, tandis qu'un anticorps reconnaissant le dit antigène est lié à la surface des particules.

**6.** Procédé selon la revendication 5, dans lequel le dit antigène est un haptène.

**7.** Procédé selon la revendication 5 ou 6, dans lequel le dit anticorps est un anticorps monoclonal.

**8.** Procédé selon l'une quelconque des revendications 5 à 7, dans lequel un antigène est lié aux fragments de gène, et un anticorps monoclonal est lié à la surface des particules.

# Fig. 1

TARGET GENE

BIOTINYLATED PCR PRIMER

PCR PRODUCT HAVING BIOTIN MOLECULES AT BOTH ENDS

ADDITION OF LATEX HAVING ANTIBIOTIN ANTIBODY BONDED THERETO

AGGLUTINATION REACTION

a. SEMICONDUCTOR LASER

b. CONDENSER LENS

c. RECEPTOR

d. BEAM STOPPER

# Fig. 2